(19) Europäisches Patentamt
European Patent Office
Office européen des brevets

(11) Veröffentlichungsnummer: 0 284 901 A1

(12) EUROPÄISCHE PATENTANMELDUNG

(21) Anmeldenummer: 88104217.0

(51) Int. Cl.4: C07D 253/06 , A01N 43/707

(22) Anmeldetag: 17.03.88

(30) Priorität: 28.03.87 DE 3710255

(43) Veröffentlichungstag der Anmeldung: 05.10.88 Patentblatt 88/40

(84) Benannte Vertragsstaaten: BE CH DE FR GB IT LI NL

(71) Anmelder: BAYER AG
Konzernverwaltung RP Patentabteilung
D-5090 Leverkusen 1 Bayerwerk(DE)

(72) Erfinder: Dickoré, Karlfried, Dr.
Nicolai-Hartmann-Strasse 19
D-5090 Leverkusen(DE)
Erfinder: Santel, Hans-Joachim, Dr.
Grünstrasse 9a
D-5090 Leverkusen(DE)
Erfinder: Schmidt, Robert Rudolf, Dr.
Im Waldwinkel 110
D-5060 Berg-Gladbach 2(DE)
Erfinder: Strang, Harry, Dr.
Unterdorfstrasse 6A
D-4000 Düsseldorf 31(DE)

(54) **Neue 3,4,6-trisubstituierte 1,2,4-Triazin-5(4H)- one.**

(57) Die Erfindung betrifft neue 3,4,6-trisubstituierte 1,2,4-Triazin-5(4H)-one der allgemeinen Formel (I)

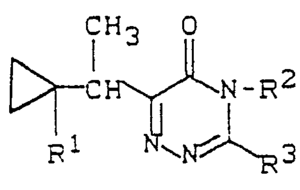

(I)

in welcher
$R^1$ für Wasserstoff oder Methyl steht,
$R^2$ für Amino, Methylamino oder Methyl steht und
$R^3$ für Methylthio, Ethylthio, Methylamino, Ethylamino, Allylamino, Dimethylamino, Methylethylamino oder Diethylamino steht,
verschiedene Verfahren zu ihrer Herstellung und ihre Verwendung als Herbizide.

EP 0 284 901 A1

## Neue 3,4,6-trisubstituierte 1,2,4-Triazin-5(4H)-one

Die Erfindung betrifft neue 3,4,6-trisubstituierte 1,2,4-Triazin-5(4H)-on-Derivate, Verfahren zu ihrer Herstellung und ihre Verwendung als Herbizide.

Es ist bereits bekannt geworden, daß bestimmte substituierte 1,2,4-Triazin-5(4H)-on-Derivate, wie beispielsweise 4-Amino-6-tert.-butyl-3-methylthio-1,2,4-triazin-5(4H)-on (Metribuzin), als Herbizide verwendet werden können (vgl. DE-PS 15 42 873; US-PS 3 671 523). In bestimmten Kulturen ist jedoch ein selektiver Einsatz der vorbekannten Triazinone nicht möglich, da es infolge der durchweg hohen herbiziden Potenz dieser Stoffgruppe auch bei bestimmten Nutzpflanzen zu Schäden kommen kann; die Verträglichkeit gegenüber den vorbekannten Triazinonen ist demnach bei verschiedenen Nutzpflanzen nicht ausreichend.

Es wurden nunmehr die neuen 3,4,6-trisubstituierten 1,2,4-Triazin-5(4H)-one der Formel (I)

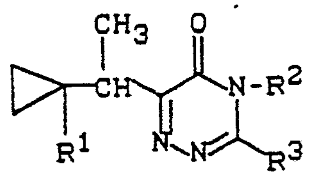

(I)

in welcher

$R^1$ für Wasserstoff oder Methyl steht,

$R^2$ für Amino, Methylamino oder Methyl steht und

$R^3$ für Methylthio, Ethylthio, Methylamino, Ethylamino, Allylamino, Dimethylamino, Methylethylamino oder Diethylamino steht,

aufgefunden.

Weiterhin wurde gefunden, daß man die neuen 3-Alkylthiotriazinonderivate der Formel (Ia)

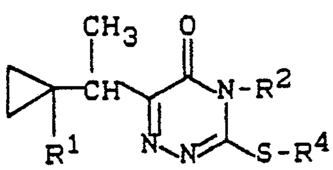

(Ia)

in welcher

$R^1$ und $R^2$ die oben angegebenen Bedeutungen haben und

$R^4$ für Methyl oder Ethyl steht,

erhält, wenn man 5-Oxo-3-thioxo-tetrahydro-1,2,4(2H,4H)-triazine der Formel (II)

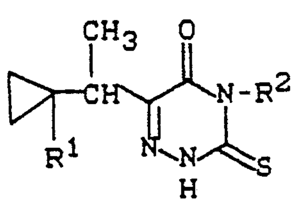

(II)

in welcher

$R^1$ und $R^2$ die oben angegebenen Bedeutungen haben,

in an sich bekannter Weise in alkalischer Lösung mittels Methyl-bzw. Ethylhalogenid (vorzugsweise -iodid oder -bromid) alkyliert (Verfahren A).

Es wurde weiterhin gefunden, daß man die neuen 3-Aminotriazinonderivate der Formel (Ib)

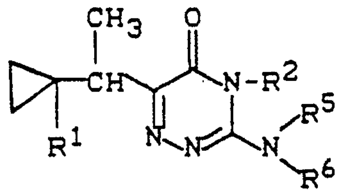 (Ib)

in welcher
$R^1$ und $R^2$ die oben angegebenen Bedeutungen haben,
$R^5$ für Wasserstoff, Methyl oder Ethyl steht und
$R^6$ für Methyl, Ethyl oder Allyl steht,
erhält, wenn man
(a) 3-Alkylthio-triazinonderivate der Formel (Ia) mit einem Amin der Formel (III)

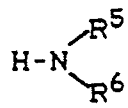 (III)

in welcher
$R^5$ und $R^6$ die obengenannten Bedeutungen haben,
gegebenenfalls in Gegenwart eines Verdünnungsmittels und gegebenenfalls in Gegenwart einer niederen aliphatischen Carbonsäure umsetzt (Verfahren B),
oder wenn man
(b) eine α-Keto-bzw. -Thioketo-carbonsäure der Formel (IV)

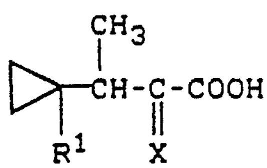 (IV)

in welcher
$R^1$ die oben angegebene Bedeutung hat und
X für Sauerstoff oder Schwefel steht,
in wäßriger Lösung mit einem Aminoguanidin-Salz der Formel (V)

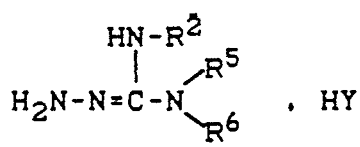 (V)

in welcher
$R^2$, $R^5$ und $R^6$ die obengenannten Bedeutungen haben und
Y für Cl, Br oder I steht,
umsetzt (Verfahren C).

Ferner wurde gefunden, daß man die neuen 3-Alkylthio-4-methylamino-triazinonderivate der Formel (Ic)

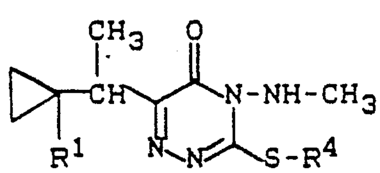 (Ic)

in welcher
$R^1$ und $R^4$ die oben angegebenen Bedeutungen haben,
auch dadurch herstellen kann, daß man ein 3-Alkylthio-4-amino-triazinonderivat der Formel (Id)

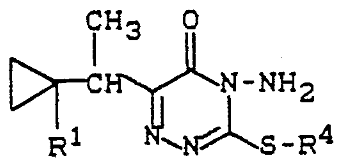

(Id)

in welcher
$R^1$ und $R^4$ die oben angegebenen Bedeutungen haben,
in an sich bekannter Weise mit einem Methylierungsmittel der Formel (VI)

$CH_3$-Z (VI)

in welcher
Z für Br, I oder $CH_3SO_4$ steht,
in Gegenwart eines Phasentransfer-Katalysators umsetzt (Verfahren D).

Außerdem wurde gefunden, daß die neuen 3,4,6-trisubstituierten 1,2,4-Triazin-5(4H)-one der Formel (I) gute her bizide, insbesondere selektiv-herbizide Eigenschaften aufweisen.

Überraschenderweise zeigen die erfindungsgemäßen Verbindungen gegenüber dem bekannten 4-Amino-6-tert.-butyl-3-methylthio-1,2,4-triazin-5(4H)-on (Metribuzin), welches konstitutionell und wirkungsmäßig eine naheliegende Verbindung ist, neben einer besseren allgemeinen herbiziden Wirkung, insbesondere eine bessere Verträglichkeit bei wichtigen Kulturpflanzen, wie bei Gerste, Mais und anderen. Die erfindungsgemäßen Wirkstoffe stellen somit eine Bereicherung der herbiziden Mittel, insbesondere der selektiven chemischen Unkrautbekämpfung dar.

Die erfindungsgemäßen Triazinonderivate sind durch die Formel (I) allgemein definiert. Besonders bevorzugte Triazinonderivate sind solche Verbindungen der Formel (I), worin $R^1$ für Wasserstoff steht, $R^2$ für Amino oder Methylamino steht und $R^3$ für Methylthio, Methylamino oder Dimethylamino steht.

Verwendet man beispielsweise 4-Amino-6-(1-cyclopropylethyl)-5-oxo-3-thioxo-tetrahydro-1,2,4(2H,4H)-triazin als Ausgangsstoff und Methyliodid als Alkylierungsmittel, so kann der Reaktionsablauf nach Verfahren A durch das folgende Formelschema wiedergegeben werden:

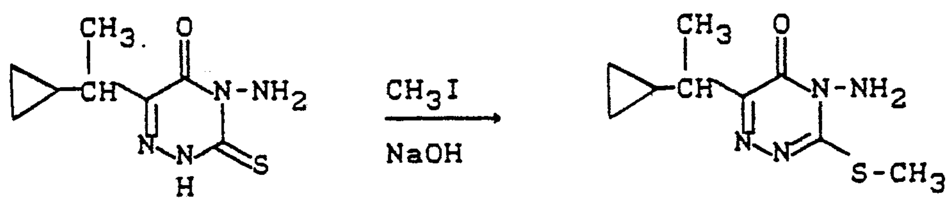


Verwendet man das so erhaltene 4-Amino-6-(1-cyclopropylethyl)-3-methylthio-1,2,4-triazin-5(4H)-on und Monomethylamin als Ausgangsstoffe, so kann der Reaktionsablauf nach Verfahren B durch das folgende Formelschema wiedergegeben werden:

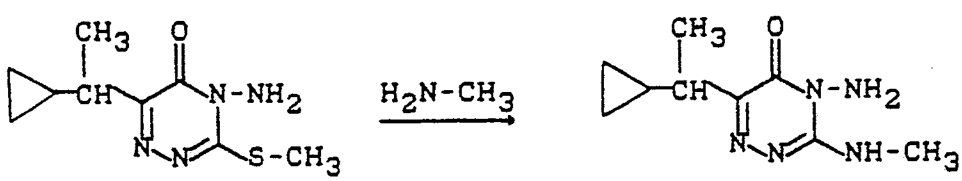


Verwendet man beispielsweise 1-Cyclopropylethyl-thionoglyoxylsäure und 1-Amino-2,2,3-trimethylguanidin-hydrochlorid als Ausgangsstoffe, so kann der Reaktionsablauf nach Verfahren C durch das folgende Formelschema wiedergegeben werden:

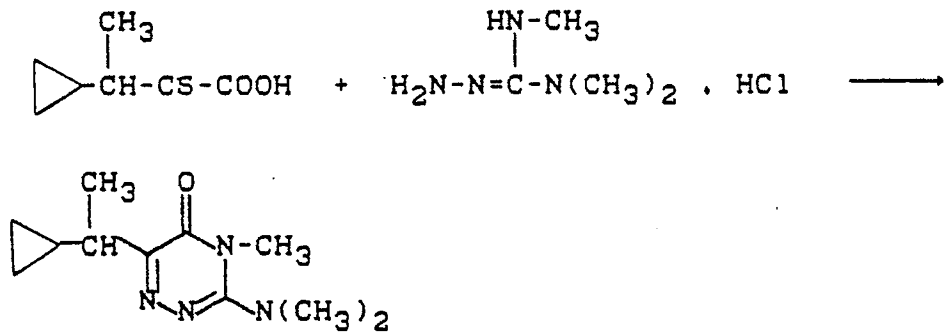

Verwendet man z.B. 4-Amino-6-(1-cyclopropylethyl)-3-methylthio-1,2,4-triazin-5-(4H)-on und Methyliodid als Ausgangsstoffe, so kann der Reaktionsablauf nach Verfahren D durch das folgende Formelschema wiedergegeben werden:

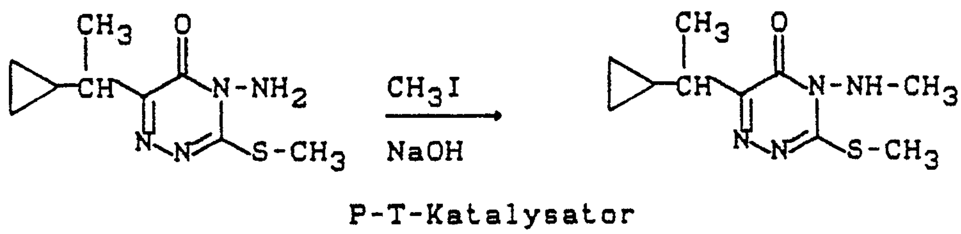


Die als Ausgangsstoffe verwendeten Verbindungen der Formel (II) sind noch nicht bekannt.

Sie können in an sich bekannter Weise hergestellt werden, indem man eine α-Keto-bzw. -Thioketo-carbonsäure der Formel (IV) mit einem Thiocarbohydrazid bzw. Thiosemicarbazid der Formel (VII) umsetzt:

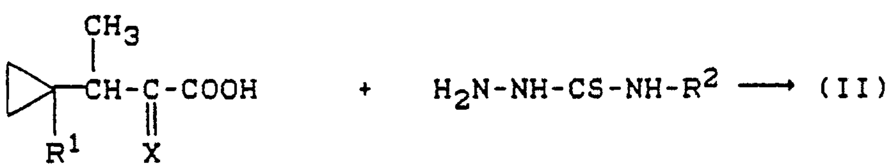

(IV) (VII)

$R^1$, $R^2$ und X haben hierbei die oben angegebenen Bedeutungen.

Die α-Keto-bzw. -Thioketo-carbonsäuren der Formel (IV) sind ebenfalls noch nicht bekannt.

Die α-Ketocarbonsäuren (IV, X = O) können z.B. durch Kondensation der bekannten Methyl-cyclopropylketone mit Hippursäure und Verseifung der erhaltenen "Azlactone" hergestellt werden (ERLENMEYER-Reaktion):

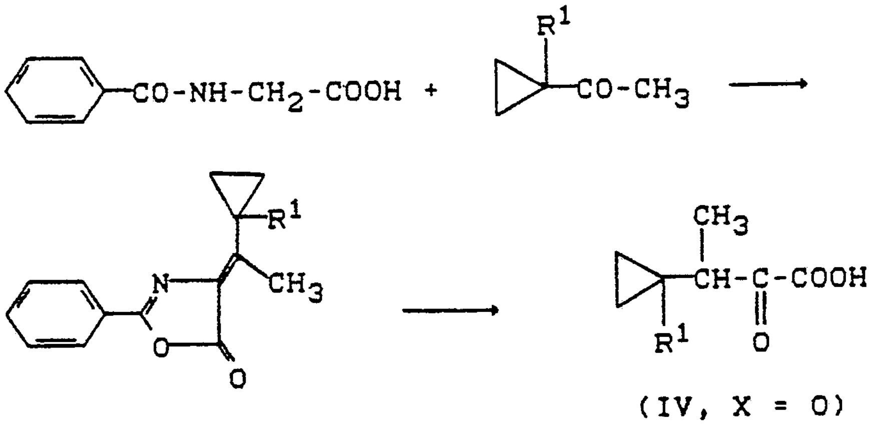

(IV, X = O)

Ein technisch gut durchführbares Verfahren zur Herstellung der α-Thioketo-carbonsäuren (IV, X = S) beruht auf der Kondensation von Methyl-cyclopropylketonen mit N-Methylrhodanin und nach folgender alkalischer Hydrolyse der so erhaltenen Alkyliden-N-methyl-rhodanine (Variante der GRÄNACHER-Reaktion). Die Kondensation der nicht isolierten α-Thioketo-carbonsäuren der Formel (IV, X = S) mit Thiosemicarbaziden bzw. Thiocarbohydraziden liefert die 6-(1-Cyclopropylethyl)-5-oxo-3-thioxo-tetrahydro-1,2,4-(2H,4H)-triazine der Formel (II):

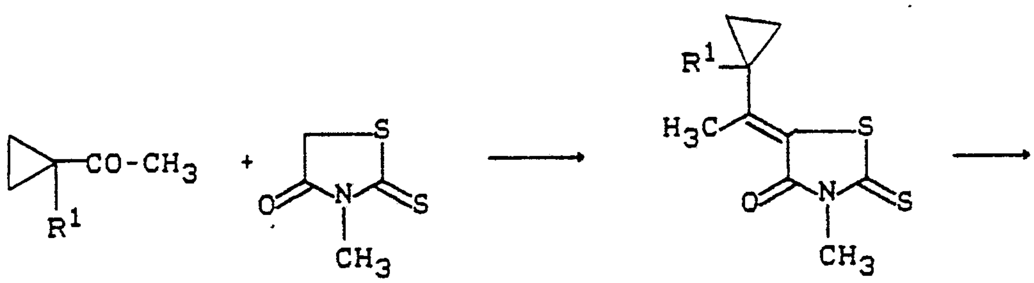

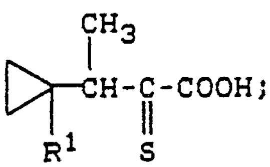;     (IV,X=S) + H-N-NH-CS-NH-$R^2$

(IV, X=S)

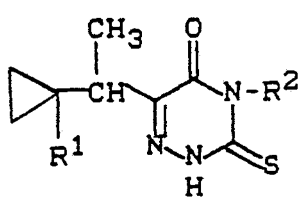

(II)

Die hierbei verwendeten Thiosemicarbazide sind bekannt; Thiocarbohydrazid ist ebenfalls bekannt.

1-Methyl-thiocarbohydrazid hingegen ist noch nicht bekannt. Es kann hergestellt werden, indem man 1-tert.-Butoxycarbonyl-1-methylhydrazin (vgl. Acta chem., scand. 22, S. 1 - 50 (1968)) mit Isopropyliden-dithiocarbazinsäure-methylester umsetzt und das erhaltene 1-tert.-Butoxycarbonyl-1-methyl-5-isopropyliden-thiocarbohydrazid hydrolysiert (s. Herstellungsbeispiele):

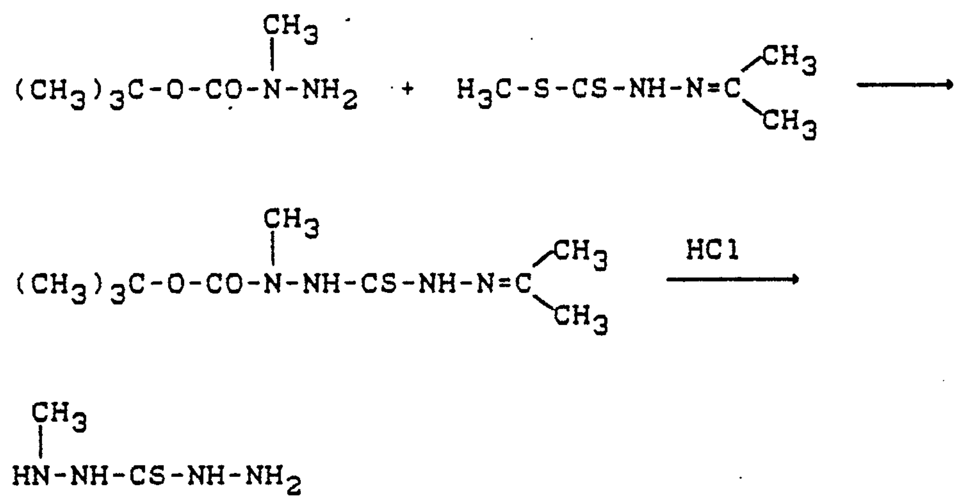


Die Alkylierung der 6-(1-Cyclopropylethyl)-5-oxo-3-thioxo-tetrahydro-1,2,4(2H,4H)-triazine der Formel (II) nach Verfahren A wird in Gegenwart einer Base durchgeführt. Hierbei werden vorzugsweise Alkalihydroxide, wie Natriumhydroxid oder Kaliumhydroxid, in wäßriger Lösung eingesetzt. Es hat sich als zweckmäßig herausgestellt, geringe Mengen eines Alkylarylpolyglykolethers als Emulgator zuzugeben. Man kann auch Alkalialkoholate, wie Natriummethylat, als Basen und Alkohole als Lösungsmittel verwenden.

Die Reaktionstemperaturen können bei der Durchführung des Verfahrens A in einem größeren Bereich variiert werden. Im allgemeinen arbeitet man zwischen 0°C und 100°C, vorzugsweise zwischen 0°C und 50°C.

Bei der Durchführung der Alkylierung nach Verfahren A setzt man auf 1 Mol des Zwischenproduktes der Formel (II) vorzugsweise 1 bis 1,5 Mol Alkylierungsmittel ein. Die Isolierung der Zwischenprodukte bzw. Endprodukte der Formel (Ia) erfolgt in üblicher Weise.

Bei der Umsetzung der 3-Alkylthio-triazinone der Formel (Ia) mit Aminen der Formel (III) nach Verfahren B kommen als Verdünnungsmittel alle inerten organischen Lösungsmittel in Frage. Hierzu gehören Kohlenwasserstoffe wie Toluol, Xylol; chlorierte aromatische Kohlenwasserstoffe wie Chlorbenzol, 1,2-Dichlorbenzol, 1,2,4-Trichlorbenzol; Ether wie Tetrahydrofuran, Dioxan; Alkohole wie Methanol, Ethanol, Propanol, Isopropanol; Amide wie N,N-Dimethylformamid, Tetramethylharnstoff oder Sulfoxide wie Dimethylsulfoxid. Vorzugsweise wird für die Umsetzung Isopropanol verwendet.

Die Reaktionstemperaturen können bei Verfahren B in einem größeren Bereich variiert werden. Im allgemeinen arbeitet man bei Temperaturen zwischen etwa 20°C und etwa 170°C, vorzugsweise zwischen 60°C und 90°C.

Die Reaktion kann drucklos wie auch bei erhöhten Drucken ausgeführt werden.

Eine besonders vorteilhafte Ausführungsform des Verfahrens B besteht darin, daß man in Gegenwart mindestens der äquimolaren Menge einer niederen aliphatischen Carbonsäure arbeitet. Vorzugsweise wird hierfür Essigsäure verwendet. Dieses Verfahren gestattet es, mit relativ geringem Dimethylamin-Überschuß auszukommen. Bei dieser Ausführungsform kann die Reaktionsgeschwindigkeit durch Zusatz einer katalytischen Menge einer organischen Sulfonsäure erhöht werden. Vorzugsweise verwendet man hierfür p-Toluolsulfosäure.

Bei der Durchführung des erfindungsgemäßen Verfahrens B nach dieser bevorzugten Verfahrensvariante setzt man auf 1 Mol eines 3-Alkylthio-triazinons der Formel (Ia) zweckmäßigerweise 1 bis 2 Mol einer niederen aliphatischen Carbonsäure, 0,01 bis 2 Mol einer organischen Sulfosäure und 1 bis 2 Mol Dimethylamin ein, erhitzt bis zum Ende der Mercaptan-Abspaltung und arbeitet anschließend auf. Dies kann z.B. in der Weise geschehen, daß man eindampft, den Rückstand mit überschüssiger wäßriger Mineralsäure verrührt und ungelöste Verunreinigungen abtrennt. Durch Zusatz einer überschüssigen Menge einer Base, z.B. Ammoniak, wird das Reaktionsprodukt in hoher Reinheit ausgefällt.

Verfahren C wird in wäßriger Lösung durchgeführt. Zweckmäßigerweise wird eine alkalische Lösung einer α-Keto-bzw. -Thioketo-carbonsäure der Formel (IV), wie sie z.B. bei der Verseifung eines Azlactons bzw. eines Alkyliden-N-methyl-rhodanins anfällt, eingesetzt.

Die Reaktionstemperaturen können bei Verfahren C gleichfalls in einem größeren Bereich variiert werden. Im allgemeinen arbeitet man bei Temperaturen zwischen etwa 50°C und etwa 100°C, vorzugsweise zwischen 65°C und 90°C.

Das Verfahren C wird im allgemeinen bei Normaldruck durchgeführt.

Bei der Durchführung des Verfahrens C setzt man auf 1 Mol einer wäßrigen Lösung des Na-Salzes der betreffenden α-Keto-bzw. -Thioketo-carbonsäure (IV) 1 bis 1,1 Mol eines Amino-guanidinium-Salzes der Formel (V) ein und erhitzt nach Zugabe von Mineralsäure, beispielsweise Salzsäure, bis zum Ende der Reaktion. Die Aufarbeitung geschieht dann in der gleichen Weise wie für Verfahren B angegeben.

Verfahren D wird im 2-Phasen-System durchgeführt. Man verwendet neben Wasser ein mit Wasser nicht mischbares Lösungsmittel wie z.B. Benzol, Toluol, Xylol, Chlorbenzol. Vorzugsweise wird Toluol verwendet. Bei der Durchführung des Verfahrens D setzt man auf 1 Mol eines 4-Amino-6-(1-cyclopropylethyl)-3-methylthio-1,2,4-triazin-5(4H)-ons 1 - 5 Mol eines Methylierungsmittels wie Methylbromid, Methyliodid oder Dimethylsulfat ein. Vorzugsweise verwendet man 2 - 4 Mol Methyliodid. Außerdem werden 0,01 bis 0,2 Mol eines quartären Ammoniumsalzes, vorzugsweise 0,08 bis 0,12 Mol Tetrabutylammoniumbromid als Phasentransferkatalysator zugesetzt.

Die Reaktionstemperaturen können bei Verfahren D ebenfalls in einem größeren Bereich variiert werden. Im allgemeinen arbeitet man bei Temperaturen zwischen 0°C und 100°C, vorzugsweise bei 20°C bis 100°C.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Es hat sich gezeigt, daß die erfindungsgemäßen Wirkstoffe im Nachauflaufverfahren für Mais gut verträglich und für Gerste besser verträglich sind als 4-Amino-6-tert.-butyl-3-methylthio-1,2,4-triazin-5(4H)-on (Metribuzin). Außerdem sind die neuen Wirkstoffe bei niedrigeren Dosierungen gegen dikotyle Unkräuter deutlich wirksamer als Metribuzin; sie können daher mit besserem Erfolg als Metribuzin zur Unkrautbekämpfung in Mais und Getreide, insbesondere in Gerste, eingesetzt werden. - Andere Kulturen, in denen die neuen Wirkstoffe selektiv eingesetzt werden können, sind: Sojabohnen, Kartoffeln, Erbsen, Luzerne.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur-und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder - schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage:

Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage:

z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist.

Für diese Mischungen können bekannte Herbizide verwendet werden, z.B. Triazine wie Atrazin, Harnstoffe wie Methabenzthiazuron, Chlortoluron oder Isoproturon, ferner Bromoxynyl und Ioxynil, oder Wuchsstoffe wie 2,4-D, MCPP und verwandte Verbindungen, oder Graminizide, wie Diclofop-methyl und das R-Enantiomere des 2-{4-[(3,5-Dichlor-2-pyridinyl)-oxy]-phenoxy}-propionsäure-(trimethylsilyl)-methylesters.

Auch eine Mischung mit anderen bekannten Wirkstoffen, wie Fungiziden, Insektiziden, Akariziden, Nematiziden, Schutztoffen gegen Vogelfraß, Wuchsstoffen, Pflanzennährstoffen und Bodenstrukturverbesserungsmitteln ist möglich.

Die Wirkstoffe können als solche in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Stäuben oder Streuen.

Die erfindungsgemäßen Wirkstoffe können sowohl vor als auch nach dem Auflaufen der Pflanzen appliziert werden. Die Anwendung wird vorzugsweise nach dem Auflaufen der Pflanzen, also im post-emergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,01 und 10 kg Wirkstoff pro ha, vorzugsweise zwischen 0,03 und 2 kg/ha.

Die erfindungsgemäßen Wirkstoffe wirken zum Teil auch als Defoliants bei Baumwolle.

Die nachfolgenden Beispiele dienen zur weiteren Erläuterung der Erfindung.

## Herstellungsbeispiele

Beispiel 1

4-Amino-6-(1-cyclopropylethyl)-3-methylthio-1,2,4-triazin-5(4H)-on

(nach Verfahren A)

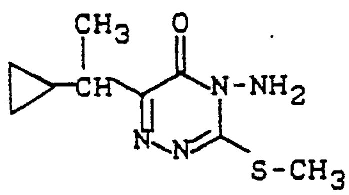

789 g (3,72 Mol) 4-Amino-6-(1-cyclopropylethyl)-5-oxo-3-thioxo-tetrahydro-1,2,4-(2H,4H)-triazin werden in einer Auflösung von 151 g (3,77 Mol) Natriumhydroxid in 4 l Wasser gelöst. Man gibt 3 ml Alkylarylpolyglykolether zu, kühlt auf 10°C ab, gibt dann 542 g (3,82 Mol) Methyliodid zu und rührt die Mischung 4 Stunden bei Raumtemperatur. Nach Absaugen, Waschen mit Wasser sowie mit Petrolether und Trocknen bei 60°C im Vakuum erhält man 748 g (89 % d.Th.) der oben bezeichneten Verbindung vom Schmelzpunkt 111 - 113°C. Die gaschromatographisch bestimmte Reinheit beträgt 99 %.

Eine aus wenig Toluol umkristallisierte Probe schmilzt bei 114 - 115°C.

In analoger Weise erhält man die folgenden Verbindungen der Formel (Ia)

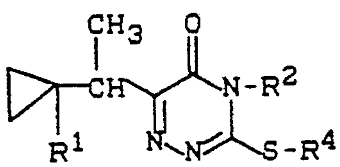

(Ia)

Tabelle 1

| Bsp. Nr. | $R^1$ | $R^2$ | $R^4$ | Fp (°C) |
|---|---|---|---|---|
| 2 | H | $NH_2$ | $C_2H_5$ | 109-110 |
| 3 | H | $CH_3$ | $CH_3$ | 64- 66 |
| 4 | H | $CH_3$ | $C_2H_5$ | 41- 43 |
| 5 | H | $NH-CH_3$ | $CH_3$ | 66- 68 |
| 6 | $CH_3$ | $NH_2$ | $CH_3$ | 132 |
| 7 | $CH_3$ | $NH_2$ | $C_2H_5$ | 74- 76 |

Beispiel 8

4-Amino-6-(1-cyclopropylethyl)-3-methylamino-1,2,4-triazin-5(4H)-on

(nach Verfahren B):

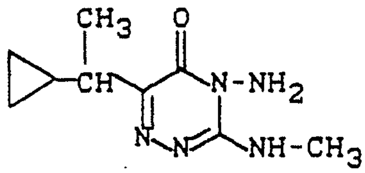

24,0 g (0,1 Mol) 4-Amino-6-(1-cyclopropylethyl)-3-ethylthio-1,2,4-triazin-5(4H)-on werden in 60 ml Isopropanol unter Zusatz von 7,2 g Essigsäure und 0,5 g p-Toluolsulfosäure gelöst und nach Einleiten von 5,0 g (0,16 Mol) Methylamin über Nacht im Ölbad von 100°C erwärmt. Nach Eindampfen verrührt man den harzartigen Rückstand mit 100 ml Wasser und 20 ml konzentrierter Salzsäure, entfernt ungelöste Anteile durch Ausschütteln mit 50 ml Methylenchlorid und fällt das Reaktionsprodukt mit 30 ml einer 25 %igen wäßrigen Ammoniak-Lösung aus: Man erhält 13,0 g (62 % d.Th.) der oben bezeichneten Verbindung vom Schmelzpunkt 125 - 127°C (aus Toluol).

In analoger Weise erhält man die folgenden Verbindungen der Formel (Ib):

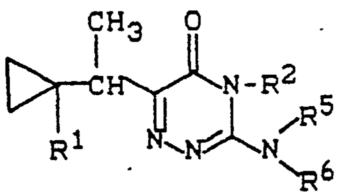

(Ib)

Tabelle 2

| Bsp. Nr. | $R^1$ | $R^2$ | $R^5$ | $R^6$ | Fp (°C)[Kp(°C/mbar)] |
|---|---|---|---|---|---|
| 9 | H | $NH_2$ | H | $C_2H_5$ | 89-90 |
| 10 | H | $NH_2$ | $CH_3$ | $CH_3$ | 88-90 |
| 11 | H | $CH_3$ | $CH_3$ | $CH_3$ | [112/0,015] |
| 12 | H | $NH-CH_3$ | $CH_3$ | $CH_3$ | [100/0,01] |
| 13 | $CH_3$ | $NH_2$ | H | $CH_3$ | 168 |
| 14 | $CH_3$ | $NH_2$ | H | $C_2H_5$ | 76-79 |
| 15 | $CH_3$ | $NH_2$ | H | $CH_2CH=CH_2$ | 86-88 |
| 16 | $CH_3$ | $NH_2$ | $CH_3$ | $CH_3$ | 70 |

Beispiel 17

6-(1-Cyclopropylethyl)-3-dimethylamino-4-methyl-1,2,4-triazin-5(4H)-on

(nach Verfahren C):

Zu einer alkalischen Verseifungslösung von Cyclopropylmethyl-methylen-N-methyl-rhodanin, die 0,1 Mol 1-Cyclopropylethyl-thiono-glyoxylsäure enthält, gibt man 15,25 g (0,1 Mol) 1-Amino-2,2,3-trimethylguanidin-hydrochlorid sowie 30 ml konzentrierte Salzsäure, rührt 1 Stunde bei Raumtemperatur und kocht anschließend 3 Stunden unter Rückfluß. Nach Abkühlen auf Raumtemperatur wird mit 50 ml Methylenchlorid ausgeschüttelt und die Methylenchlorid-Phase verworfen. Die wäßrige Phase versetzt man mit 50 ml einer 25 %igen wäßrigen Ammoniak-Lösung, nimmt das ölige Reaktionsprodukt in Methylenchlorid auf und destilliert den Eindampfungsrückstand im Vakuum.

Man erhält 16,0 g der obengenannten Verbindung in Form eines hellgelben Öls vom Siedepunkt 112°C bei 0,015 mbar (72 % d. Th.), identisch mit einem nach Verfahren B hergestellten Produkt (vgl. Beispiel 11).

Beispiel 18

6-(1-Cyclopropylethyl)-4-methylamino-3-methylthio-1,2,4-triazin-5(4H)-on

(nach Verfahren D):

Zu 100 ml 50 %iger wäßriger Natronlauge gibt man unter starkem Rühren 100 ml Toluol sowie 56,5 g (0,25 Mol) 4-Amino-6-(1-cyclopropylethyl)-3-methylthio-1,2,4-triazin-5(4H)-on, danach 89,5 g (0,63 Mol) Methyliodid und 8,5 g (0,025 Mol) Tetrabutylammoniumbromid. Die Emulsion wird bis zum Abklingen der exothermen Reaktion (Temperaturanstieg auf 50°C) stark gerührt. Man saugt die Mischung bei 20°C durch eine Glasfritte, trennt die Phasen und dampft die organische Phase ein. Der Rückstand wird aus einem Volumen-Teil Methyl-tert.-butylether umkristallisiert. Man erhält 32,9 g der oben bezeichneten Verbindung vom Schmelzpunkt 66 - 68°C, identisch mit einem nach Verfahren A hergestellten Produkt (vgl. Beispiel 5).

Herstellung der Ausgangsprodukte

4-(Cyclopropyl-methyl-methylen)-N-methyl-rhodanin

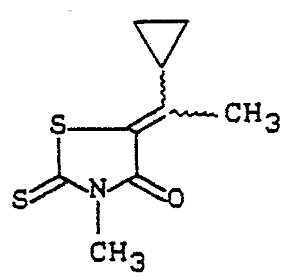

867 g (5,9 Mol) N-Methylrhodanin und 555 g (6,54 Mol) Cyclopropyl-methyl-keton werden unter Zusatz von 48 g (0,6 Mol) Ammonacetat und 72 g (1,2 Mol) Essigsäure in 1 l Benzol am Wasserabscheider unter Rückfluß bis zum Ende der Wasserauskreisung gekocht (14 Stunden). Bei 60°C verrührt man mit 1 l Wasser, läßt auf Raumtemperatur abkühlen, saugt ab und kristallisiert das feuchte Rohprodukt aus 4,5 l Ethanol um. Man erhält 1118 g (89 % d. Th.) der oben bezeichneten Verbindung als E/Z-Gemisch im Verhältnis 60:40, Schmelzbereich: 85 - 97°C.

Analog wird hergestellt:

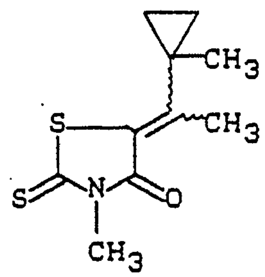

Fp: 40 - 47°C als E/Z-Gemisch; das reine E-Isomere schmilzt bei 54°C (aus Methanol).

4-Amino-6-(1-cyclopropylethyl)-5-oxo-3-thioxo-tetrahydro-1,2,4-(2H,4H)-triazin

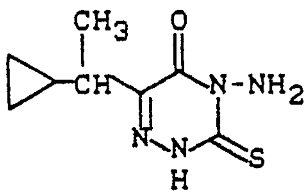

882 g (4,14 Mol) 4-(Cyclopropyl-methyl-methylen)-N-methyl-rhodanin (als E/Z-Gemisch) werden in eine Auflösung von 712 g (17,8 Mol) Natriumhydroxid in 4,4 l Wasser eingetragen. Die Mischung wird nach Zusatz von 7 ml Alkylarylpolyglykolether bei 70°C bis zur vollständigen Lösung (ca. 16 Stunden) gerührt. Man gibt sodann 438 g (4,14 Mol) Thiocarbohydrazid sowie 115 ml Decanol-1 zu, rührt eine Stunde bei 70°C und läßt rasch 1525 ml Essigsäure zutropfen, wobei das Reaktionsprodukt ausfällt. Man rührt noch 2 Stunden bei 80°C, läßt auf Raumtemperatur abkühlen, saugt ab, wäscht mehrfach mit Wasser sowie mit Petrolether und trocknet bei 70°C. Man erhält 725 g (82,6 % d.Th.) der oben bezeichneten Verbindung vom Schmelzpunkt 142 - 143°C.

In ähnlicher Weise werden hergestellt:

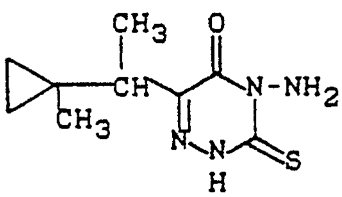

Fp: 196-198°C

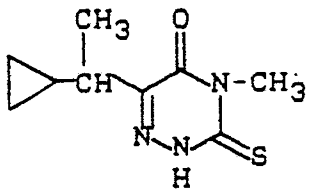

Fp: 133-134° C

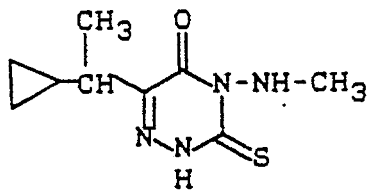

(Harz)

1-Methyl-thiocarbohydrazid

142 g (1,17 Mol) Dithiocarbazinsäure-methylester werden in 300 ml Aceton eingetragen. Nach vorübergehender Lösung und Temperaturanstieg auf 45°C kristallisieren 168 g (89 % d.Th.) Isopropyliden-dithiocarbazinsäuremethylester vom Schmelzpunkt 115°C aus.

162 g (1 Mol) dieser Verbindung werden mit 190 g (1,2 Mol) 92 %igem 1-tert.-Butoxycarbonyl-1-methylhydrazin vermischt und 8 Stunden im Ölbad von 110°C gerührt, wobei allmählich Methylmercaptan abgespalten wird. Das kalt ausgefallene 1-tert.-Butoxycarbonyl-1-methyl-5-isopropyliden-thiocarbohydrazid wird abgesaugt und mit Toluol gewaschen. Man erhält 139 g (53,5 % d.Th.) vom Schmelzbereich 165 - 172°C (Zers.).

130 g (0,5 Mol) dieser Verbindung löst man in 460 ml halbkonzentrierter Salzsäure, rührt 3 Stunden bei Raumtemperatur und dampft ein. Der Rückstand wird in 460 ml Wasser gelöst, mit 25 %iger Ammoniaklösung neutralisiert und eingedampft. Der trockene Rückstand wird mehrfach mit Methanol ausgekocht. Aus dem filtrierten Methanol-Extrakt erhält man nach dem Eindampfen 52,8 g (88 % d.Th.) 1-Methyl-thiocarbohydrazid als steifes Öl.

Beispiel A

Post-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welches eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)

100 % = totale Vernichtung

Die erfindungsgemäßen Verbindungen zeigen in diesem Test eine hervorragende herbizide Wirksamkeit. Beispielsweise zeigen in diesem Test die Verbindungen gemäß den Her stellungsbeispielen 1, 5 (bzw. 18), 8, 10, 12 und 16 im Vergleich mit dem nächstliegenden Stand der Technik (Metribuzin) eine wesentlich stärkere Wirkung gegen Problemunkräuter, wie z.B. Cassia, Amaranthus, Polygonum, Ipomoea und Solanum, bei gleichzeitig sehr guter Verträglichkeit gegenüber Kulturpflanzen, wie z.B. Mais und Gerste.

Beispiel B

Pre-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)

100 % = totale Vernichtung

In diesem Test zeigen die erfindungsgemäßen Verbindungen der Formel (I) ebenfalls sehr gute Wirkung.

**Ansprüche**

1) 3,4,6-Trisubstituierte 1,2,4-Triazin-5(4H)-one der allgemeinen Formel (I)

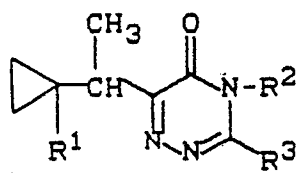

(I)

in welcher

$R^1$ für Wasserstoff oder Methyl steht,

$R^2$ für Amino, Methylamino oder Methyl steht und

$R^3$ für Methylthio, Ethylthio, Methylamino, Ethylamino, Allylamino, Dimethylamino, Methylethylamino oder Diethylamino steht.

2) Triazinon-Derivate der allgemeinen Formel (I) gemäß Anspruch 1, dadurch gekennzeichnet, daß darin

$R^1$ für Wasserstoff steht,

$R^2$ für Amino oder Methylamino steht und

$R^3$ für Methylthio, Methylamino oder Dimethylamino steht.

3) Verfahren zur Herstellung von 3-Alkylthiotriazinon-Derivaten der Formel (Ia)

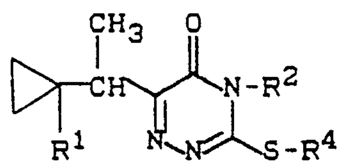

(Ia)

in welcher

$R^1$ für Wasserstoff oder Methyl steht,

$R^2$ für Amino, Methylamino oder Methyl steht und

$R^4$ für Methyl oder Ethyl steht,
dadurch gekennzeichnet, daß man 5-Oxo-3-thioxotetrahydro-1,2,4(2H,4H)-triazine der Formel (II)

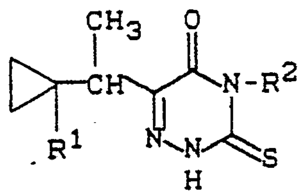

(II)

in welcher
$R^1$ und $R^2$ die oben angegebenen Bedeutungen haben,
in an sich bekannter Weise in alkalischer Lösung mittels Methyl-bzw. Ethylhalogenid (vorzugsweise -iodid oder -bromid) alkyliert.

4) Verfahren zur Herstellung von 3-Amino-triazinon-Derivaten der Formel (Ib)

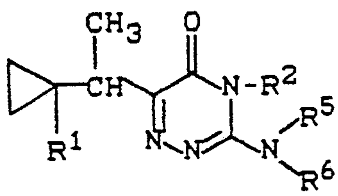

(Ib)

in welcher
$R^1$ für Wasserstoff oder Methyl steht,
$R^2$ für Amino, Methylamino oder Methyl steht und
$R^5$ für Wasserstoff, Methyl oder Ethyl steht und
$R^6$ für Methyl, Ethyl oder Allyl steht,
dadurch gekennzeichnet, daß man
(a) 3-Alkylthio-triazinonderivate der Formel (Ia) gemäß Anspruch 3 mit einem Amin der Formel (III)

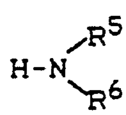

(III)

in welcher
$R^5$ und $R^6$ die obengenannten Bedeutungen haben,
gegebenenfalls in Gegenwart eines Verdünnungsmittels und gegebenenfalls in Gegenwart einer niederen aliphatischen Carbonsäure umsetzt,
oder daß man
(b) eine α-Keto-bzw. -Thioketo-carbonsäure der Formel (IV)

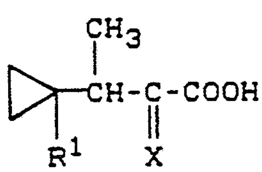

(IV)

in welcher
$R^1$ die oben angegebene Bedeutung hat und
X für Sauerstoff oder Schwefel steht,
in wäßriger Lösung mit einem Aminoguanidin-Salz der Formel (V)

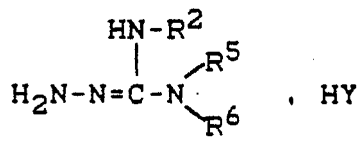

(V)

in welcher
$R^2$, $R^5$ und $R^6$ die obengenannten Bedeutungen haben und
Y für Cl, Br oder I steht,
umsetzt.

5) Verfahren zur Herstellung von 3-Alkylthio-4-methylamino-triazinon-Derivaten der Formel (Ic)

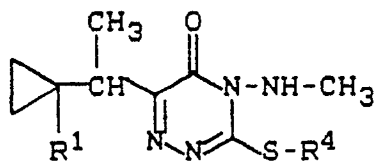

(Ic)

in welcher
$R^1$ für Wasserstoff oder Methyl steht und
$R^4$ für Methyl oder Ethyl steht,
dadurch gekennzeichnet, daß man 3-Alkylthio-4-amino-triazinon-Derivate der Formel (Id)

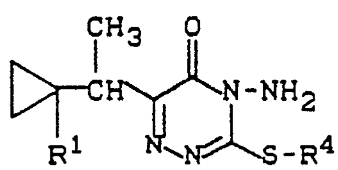

(Id)

in welcher
$R^1$ und $R^4$ die oben angegebenen Bedeutungen haben,
in an sich bekannter Weise mit einem Methylierungsmittel der Formel (VI)

$CH_3$-Z (VI)

in welcher
Z für Br, I oder $CH_3SO_4$ steht,
in Gegenwart eines Phasentransfer-Katalysators umsetzt.

6) Herbizide Mittel, gekennzeichnet durch einen Gehalt an mindestens einem 3,4,6-trisubstituierten 1,2,4-Triazin-5(4H)-on der allgemeinen Formel (I) gemäß Anspruch 1.

7) Verfahren zur Bekämpfung von unerwünschtem Pflanzenwachstum, dadurch gekennzeichnet, daß man 3,4,6-trisubstituierte 1,2,4-Triazin-5(4H)-one der allgemeinen Formel (I) gemäß Anspruch 1 auf die Pflanzen oder ihren Lebensraum einwirken läßt.

8) Verwendung von 3,4,6-trisubstituierten 1,2,4-Triazin-5(4H)-onen der allgemeinen Formel (I) gemäß Anspruch 1 zur Bekämpfung von Pflanzenwachstum.

9) Verfahren zur Herstellung von herbiziden Mitteln, dadurch gekennzeichnet, daß man 3,4,6-trisubstitu ierte 1,2,4-Triazin-5(4H)-one der allgemeinen Formel (I) gemäß Anspruch 1 mit Streckmitteln und/oder oberflächenaktiven Mitteln vermischt.

Europäisches Patentamt

# EUROPÄISCHER RECHERCHENBERICHT

Nummer der Anmeldung

EP 88 10 4217

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE-A-2 417 511 (BAYER AG)<br>* Ansprüche *<br>--- | 1,3,4,6-9 | C 07 D 253/06<br>A 01 N 43/707 |
| A | DE-A-2 856 750 (CIBA-GEIGY AG/DEGUSSA)<br>* Ansprüche 1-3; Seite 3 - Seite 5, Zeile 20 *<br>--- | 1,3,6,8 | |
| A | DE-A-3 240 308 (BAYER AG)<br>* Ansprüche 1,2,5-7 *<br>--- | 1,4,6-9 | |
| A | EP-A-0 130 518 (BAYER AG)<br>* Ansprüche 1,3,5-7 *<br>--- | 1,6-9 | |
| D,A | DE-B-1 542 873 (BAYER AG)<br>* Anspruch; Spalten 7-24, Tabellen * & US - A - 3 671 523 (Cat. D,A) *<br>----- | 1,6,8 | |
| | | | RECHERCHIERTE SACHGEBIETE (Int. Cl.4) |
| | | | C 07 D 253/00 |

Der vorliegende Recherchenbericht wurde für alle Patentansprüche erstellt

| Recherchenort | Abschlußdatum der Recherche | Prüfer |
|---|---|---|
| BERLIN | 24-06-1988 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)